# EUROPEAN PATENT APPLICATION

(11) **EP 4 265 198 A1**
(43) Date of publication of application: **25.10.2023**
(21) Application number: 21905844.3
(22) Date of filing: 17.12.2021
(51) Int. Cl.: A61B 17/076, A61B 17/10, A61B 17/128

(54) **VALVE CLIP REMOVAL DEVICE**

(30) Priority: 18.12.2020 CN 202011507493
(71) Applicant: Enlight Medical Technologies (Shanghai) Co., Ltd., Shanghai 201318 (CN)
(72) Inventor: BIAN, Yingnan, Shanghai 201318 (CN); GAO, Yi, Shanghai 201318 (CN); WANG, Xingzhao, Shanghai 201318 (CN)
(74) Representative: Fuchs Patentanwälte Partnerschaft mbB
(86) International application number: PCT/CN2021/139340
(87) International publication number: WO 2022/127921

(57) **Abstract**

A valve clip removal device, comprising: an outer tube (100), a capture device (200), and a cutter (300), wherein the capture device (200) comprises one or more capture heads (210); the outer tube (100) is provided with a recovery cavity (110), and the capture heads (210) are arranged at a distal end of the outer tube (100); the capture heads (210) can extend out, in a direction away from the distal end of the outer tube (100), and grasp a valve clip , and can also be withdrawn in a direction towards the distal end of the outer tube (100) and capture the valve clip in the recovery cavity (110) of the outer tube (100); a distal portion of the cutter (300) is arranged around the interior of the recovery cavity (110) of the outer tube (100); and the cutter (300) is used to cut off the valve clip captured in the recovery cavity (110) of the outer tube (100). By means of the removal device, an implant can be separated or removed via a catheter, for the preparation of a patient before further valve replacement. In addition, the removal device is simple and reliable, and can be operated conveniently and quickly.

## Description

### TECHNICAL FIELD

The present disclosure disclosed herein relates to the field of medical device technology, and in particular to a valve clip removal device.

### BACKGROUND

The incidence of heart valve disease is associated with increasing age. With the advent of aging society, the incidence has increased significantly. In which, mitral regurgitation (MR) is a common valvular disease. Mitral regurgitation is the abnormal flow of blood from the left ventricle into the left atrium during systole. Severe mitral regurgitation can lead to symptoms such as palpitations, dyspnea, and chest tightness. The annual mortality rate for those with severe heart failure is upwards of 30%. Patients with mitral regurgitation are usually treated with surgical valve repair or valve replacement. With the development of transcatheter valve technology, transcatheter mitral valve clip is also applied to improve regurgitation.

The principle of the transcatheter mitral valve clip is to simulate the edge to edge technique used in surgical repair surgery. The catheter introduces on or more mitral valve clips through the right femoral vein. The anterior and posterior leaflets of the mitral valve are held at each end of the clip to increase the coaptation area of the anterior and posterior leaflets and reduce the mitral regurgitation. The MitraClip is currently widely used. Generally, this type of clip is made of metal and covered with mesh on the outside. However, this type of mitral valve clip also has obvious deficiency. After the implantation of the mitral valve clip, if it is to be removed, it usually requires a surgical thoracotomy which brings huge risks to patients. In some patients, mitral valve regurgitation is still significant after mitral valve clip implantation, and the mitral valve clip needs to be reimplanted. There are also some patients with mitral regurgitation recurring after several years of implantation, and there is not much choice at this time. The choice includes the transcatheter mitral annular contraction or the transcatheter mitral valve replacement. The use of a mitral valve prosthesis to completely replace the native valve can completely eliminate the problem of mitral regurgitation. If such patients opt for transcatheter mitral valve replacement, the formerly implanted mitral valve clip impedes the release of the artificial mitral valve. Therefore, it is necessary to remove the valve clip, namely separates the valve clip from the anterior and/or posterior leaflets of the mitral valve it holds.

However, the system in prior art that approaches the relative leaflets in the heart valve to remove the implanted clip has the following shortcomings: (a) In design operations, whether using magnetic components for connecting or ring structures at the free end of cutting components, they will be affected by the impact of valve leaflets, chordae tendineae, and blood flow, resulting in incorrect connecting or failure of connecting; (b) In addition, the system in prior art may generate tissue debris during cutting, and possible embolism problems were not considered.

Therefore, it is necessary to provide a valve clip removal device in this field, which can overcome the above shortcomings and separate the valve clip from the anterior and/or posterior leaflets of the mitral valve that it holds, and can further separate or remove the implant through a catheter, preparing for the patients who need further valve replacement surgery.

### SUMMARY

The purpose of this disclosure is to provide a valve clip removal device, which can separate the valve clip from the anterior and/or posterior leaflets of the mitral valve that it holds, and can further separate or remove the implant through a catheter. The removal device is simple and reliable, and can be operated conveniently and quickly, preparing for the patients who need further valve replacement surgery.

The first aspect of this disclosure provides a valve clip removal device, comprising an outer tube, a capture device, and a cutter. The capture device comprises one or more capture heads provided at a distal end, and the cutter comprises a cutting component provided at a distal end.

The outer tube has a retrieve cavity, the capture head(s) is movably provided at a distal end of the outer tube, and the capture heads are configured to capture a valve clip; The cutting component is movably provided in the cavity of the retrieve cavity of the outer tube, and is configured to separate the captured valve clip from at least part of a native valve tissue held by the valve clip.

In another preferred embodiment, the capture device further comprises a control component and a capture handle, the control component passes through the retrieve cavity of the outer tube, and a distal end of the control component is connected to the capture head, a proximal end of the control component is connected to the capture handle, which drives the capture heads to move by controlling the control component to achieve capture of the valve clip.

In another preferred embodiment, a proximal end of the capture head comprises an accommodating cavity, which is configured to accommodate the valve clip to achieve the capture of the valve clip, and to retrieve the valve clip to be cut off into the retrieve cavity.

In another preferred embodiment, the capture head is hemispheric, 1/4 spherical, pyramidal, cylindrical, conical or tetrahedral in shape.

In another preferred embodiment, the cutter further comprises a blade and a cutting handle, a proximal end of the blade is connected to the cutting handle, and a distal end of the blade is connected to the cutting component, the cutting handle is configured to control the movement of the cutting component and the blade relative to the outer tube, so as to separate the captured valve clip from at least part of the native valve tissue held by the valve clip.

In another preferred embodiment, at least a distal end of the control component is disposed between a sidewall of the retrieve cavity and the blade in the radial direction.

In another preferred embodiment, the cutting component comprises a cutting head for cutting and separating the captured valve clip from at least part of the native valve tissue held by the valve clip.

In another preferred embodiment, the cutting head is circular in shape, or the cutting head is in a plurality and arranged circumferentially around an axis of the outer tube.

In another preferred embodiment, the cutting component comprises needle or blade shaped electrodes connected to a high-frequency AC power supply, the electrodes are configured to separate the captured valve clip from at least part of the native valve tissue held by the valve clip by electrocoagulation, or the cutting component comprises an electric heating wire electrically connected to a power supply, and the electric heating wire is configured to separate the captured valve clip from at least part of the native valve tissue held by the valve clip by burning.

In another preferred embodiment, the proximal end of the capture head is matched to the distal end of the outer tube so as to form a closed space by the capture head and the retrieve cavity of the outer tube when the capture head takes the captured valve clip into the retrieve cavity of the outer tube.

In another preferred embodiment, the capture head comprises a mesh structure or a claw-like structure for capturing and fastening the valve clip.

In another preferred embodiment, the proximal end of the capture head comprises an accommodating cavity, which is configured to accommodate the valve clip to achieve the capture of the valve clip, and to retrieve the valve clip to be cut off into the retrieve cavity.

The other aspect of this disclosure provides a valve clip removal device, comprising an outer tube, a capture device, and a cutter. The capture device comprises one or more capture heads.

The outer tube has a retrieve cavity, the capture heads are disposed at a distal end of the outer tube, and the capture heads can extend in a direction away from the distal end of the outer tube and capture a valve clip, and can also be retracted in a direction towards the distal end of the outer tube and take the captured valve clip into the retrieve cavity of the outer tube; the distal end of the cutter is disposed around the inner wall of the retrieve cavity of the outer tube, and the cuter is configured to cut off the captured valve clip in the retrieve cavity of the outer tube.

In another preferred embodiment, the capture device further comprises a control component and a capture handle, the control component is disposed in the retrieve cavity of the outer tube, the distal end of the control component is connected to the capture head, and the proximal end of the control component is connected to the capture handle, which controls the control component to drive the capture head to move, thereby achieving the capture and retracting of the valve clip.

In another preferred embodiment, the cutter further comprises a cutting head, a blade and a cutting handle which is configured to control the axial and circumferential movement of the cutting head and the blade along the outer tube, thereby cutting the valve clip.

In another preferred embodiment, the distal end of the blade is connected to the cutting head, the proximal end of the blade is connected to the cutting handle.

In another preferred embodiment, the capture head is provided with a groove, which is configured to accommodate part of the distal end of the cutting head to form a closed space by the capture head and the inner cavity of the outer tube when the capture head takes the captured valve clip into the retrieve cavity of the outer tube.

In another preferred embodiment, the control component is disposed between the inner wall of the outer tube and the blade.

In another preferred embodiment, the cutting head and the distal end of the blade is disposed in the retrieve cavity of the outer tube.

In another preferred embodiment, the capture head is hemispheric, 1/4 spherical, pyramidal, cylindrical, conical or tetrahedral in shape.

In another preferred embodiment, the capture head attaches to the distal end of the outer tube when the capture head takes the captured valve clip into the retrieve cavity of the outer tube, thereby forming a closed space by the capture head and the retrieve cavity of the outer tube.

In another preferred embodiment, the cutting of the cutter is completed in the inner cavity of the outer tube.

In another preferred embodiment, in the axial direction of the outer tube, the capture handle and the cutting handle are disposed far away from the distal end of the outer tube, and the capture handle is disposed above the cutting handle.

In another preferred embodiment, the capture head is provided with a radiopaque marker(s) (or developer) for marking the position of the capture head.

In another preferred embodiment, the cutting head is provided with a radiopaque marker(s) for marking the position of the cutting head.

In another preferred embodiment, the distal end of the outer tube is provided with a radiopaque marker(s) for marking the position of the distal end of the outer tube.

In another preferred embodiment, the cutting head, the blade and the cutting handle can be formed integrally.

In another preferred embodiment, the cutting head, the blade and the cutting handle are independently connected.

In another preferred embodiment, any two of the cutting head, the blade and the cutting handle are formed in one piece and connected to the remaining third component.

In another preferred embodiment, the cutting head is flat mouthed, serrated, straight toothed, or "I" shaped.

It should be understood that within the scope of the present disclosure, the above-mentioned technical features of the disclosure and the specific technical features described below (according to the embodiments) can be combined with each other to form new or preferred technical solutions. Due to space limitations, it will not go into detail here.

### BRIEF DESCRIPTION OF THE DRAWINGS

In order to provide a clearer explanation of the embodiments of the present disclosure or the technical solutions in the prior art, a brief introduction will be given to the drawings required in the description of the embodiments or prior art. It should be understood that the drawings in the following description are only some implementation examples of the present invention. Ordinary technical personnel in this field can also obtain other implementation examples based on these drawings without creative labor.
Fig. 1 is a structural schematic diagram of a valve clip removal device according to an embodiment of the present disclosure.
Fig. 2 is a structural schematic diagram of a capture device of the valve clip removal device according to an embodiment of the present disclosure.
Fig. 3 is a structural schematic diagram of a cutter of the valve clip removal device according to an embodiment of the present disclosure.

In each drawing, the markings are as follows:
100-outer tube
110-retrieve cavity
200-capture device
210-capture head
2101-accommodating cavity
21 1-groove
220-control component
230-capture handle
300-cutter
310-cutting head
320-blade
330-cutting handle

### DETAILED DESCRIPTION

The inventor, through extensive and in-depth research, has for the first time disclosed a valve clip removal device. Through clever structural design, the removal device not only achieves the removal of the valve clip, but also has a simple and reliable structure of the capture device and can be operated conveniently and quickly. Moreover, the structure of the cutter of the valve clip removal device is simple and reliable, and it doesn't need to introduce new cutter when cutting the valve. During the cutting process, it is not easy to form tissue debris which causes embolism.

### Terms

As used herein, the terms "valve clip" and "clip" are interchangeable;

As used herein, the terms "distal (end)" and "distal part" refer to the end near the valve clip when using the valve clip removal device, and also refer to the end far from the operator;

As used herein, the terms "proximal (end)" and "proximal part" refer to the end far from the valve clip when using a valve clip removal device, and also refer to the end near the operator;

As used herein, the term "blade" refers to a cutting body that is connected to the cutting component of the cutter at the distal end and the cutting handle of the cutter at the proximal end, the terms "blade" and "cutting body" are interchangeable.

It should be noted that in the disclosure documents of this application, relational terms such as first and second are only used to distinguish one entity or operation from another entity or operation, and do not necessarily require or imply any actual relationship or order between these entities or operations. Moreover, the terms "comprising", "including", or any other variation thereof are intended to encompass non-exclusive inclusion, such that a process, method, item, or device that includes a series of elements not only includes those elements, but also other elements that are not explicitly listed, or also include elements inherent in such a process, method, item, or device. Without further limitations, the element limited by the statement 'comprising a' does not exclude the existence of another identical element in the process, method, item, or device that includes the element. In the disclosure documents of this application, if it is mentioned that a certain act is performed based on a certain element, it means that at least the act is performed based on that element, which includes two situations: only performing the act based on that element, and performing the act based on that element and other elements. Multiple, one and/or more expressions include 2 and more than 2 or more.

In the present disclosure, all directional indications (such as up, down, left, right, front, rear, etc.) are only used to explain the relative position relationship, motion situation, etc. between components in a specific posture (as shown in the attached drawings). If the specific posture changes, the directional indication also changes accordingly.

In the following description, many technical details have been proposed to help readers better understand this disclosure. However, ordinary technical personnel in this art can understand that even without these technical details and various changes and modifications based on the following implementation methods, the technical solution claimed for protection in this application can still be achieved.

In order to clarify the purpose, technical solution, and advantages of the present disclosure, a further detailed description of the implementation method of the present disclosure will be provided below in conjunction with the attached drawings.

The present disclosure provides a valve clip removal device, which comprises an outer tube, a capture device, and a cutter. The capture device includes one or more capture heads provided at the distal end, and the cutter includes cutting component provided at the distal end.

The outer tube has a retrieve cavity, the capture head(s) is movably provided at the distal end of the outer tube, and the capture head(s) is used to capture the valve clip. The cutting component is movably disposed in a cavity of the retrieve cavity of the outer tube, and is used to remove the captured valve clip, which is to separate the captured valve clip from at least part of the native valve tissue held by the valve clip.

The native valve tissue held by the valve clip here can be the native valve leaflet, the chordae tendineae, or other components of the native valve. This application is not limited in this respect.

The following takes the removal of the valve clip for clipping a mitral valve native leaflet as an example, but the protection scope of the invention is not limited to the embodiment, for example, it can also be used for the removal of the valve clip for clipping the tricuspid valve tissue.

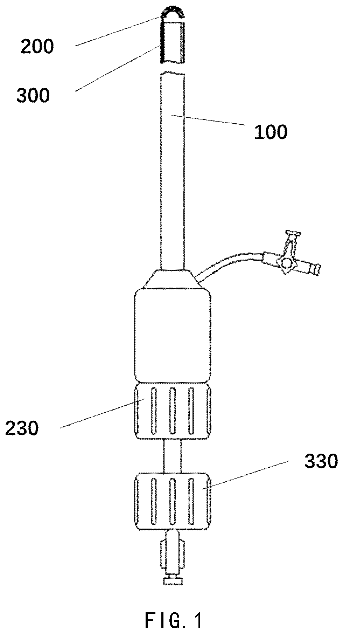

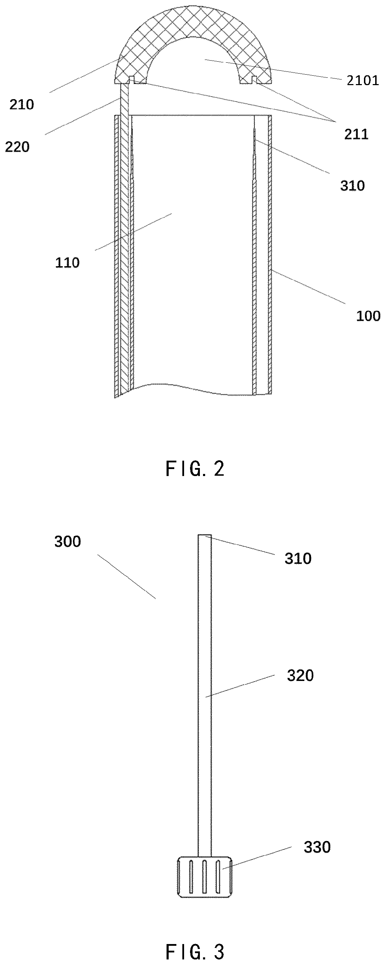

Referring to Figs. 1-3, the valve clip removal device of this embodiment comprises an outer tube 100, a capture device 200, and a cutter 300. The outer tube 100 is provided with a retrieve cavity 110. The capture device 200 includes one or more capture heads 210, a control component 220, and a capture handle 230. The cutter 300 includes a cutting component, a blade 320, and a cutting handle 330.

The capture head 210 of the capture device 200 is provided at the distal end of the outer tube 100 and can move freely relative to the outer tube 100, that is, it can move along the axis direction of the outer tube 100 and/or rotate around the axis of the outer tube 100. The capture head 210 is used to capture the valve clip. In this embodiment, the number of the capture head 210 is one, and the capture head 210 is in a hemispherical shape. The capture head 210 can take on other shapes, such as 1/4 spherical, pyramidal, cylindrical, conical or tetrahedral, which is not limited in this embodiment.

Furthermore, the proximal end of the capture head 210 includes an accommodating cavity 2101, which is used to accommodate the valve clip to achieve capturing of the valve clip, and to retrieve the valve clip to be removed into the retrieve cavity 110. In this embodiment, the accommodating cavity 2101 is hemispherical in shape to increase the surface area that can be in contact with the valve clip. In other embodiments, the accommodating cavity 2101 can also be of other shapes, such as pyramidal, cylindrical, conical, or tetrahedral.

Furthermore, the proximal end of the capture head 210 is matched with the distal end of the outer tube 100. In this way, when the capture head 210 takes the captured the valve clip into the retrieve cavity 110 of the outer tube 100, the capture head 210 forms a closed space with the retrieve cavity 110 of the outer tube 100.

Furthermore, a radiopaque marker(s) is provided on the capture head 210 to show the position of the capture head 210.

In this embodiment, the control component 220 passes through the retrieve cavity 110 of the outer tube 100, the distal end of the control component 220 is connected to the capture head 210, and the proximal end of the control component 220 is connected to the capture handle 230. The operator operates the capture handle 230, which drives the capture head 210 through the control component 220 to achieve the capture of the valve clip.

The cutting component is movable disposed in the cavity of the retrieve cavity 110 of the outer tube 100, which can move along the axis direction of the outer tube 100 and/or rotate around the axis of the outer tube 100. The cutting component is used to separate the captured valve clip from at least part of the native valve tissue held by the valve clip. In this embodiment, the cutting component is used to separate the captured valve clip from at least part of the mitral valve native leaflet held by the valve clip.

Correspondingly, the proximal end of the blade 320 is connected to the cutting handle 330, and the distal end of the blade 320 is connected to the cutting component. The cutting handle 330 is used to control the movement of the cutting component and the blade 320 relative to the outer tube 100, thereby separating the captured valve clip from at least part of the native valve tissue held by the valve clip.

Preferably, at least the distal end of the control component 220 is disposed between the inner wall of the outer tube 100 and the blade 320. Therefore, the distal part of the control component 220 of the capture device 200 and the cutting component and blade 320 of the cutter 300 are all accommodated in the retrieve cavity of the outer tube, and the separation and retrieve of the valve clip can be completed in the retrieve cavity 110 of the outer tube. For example, the proximal part of control component 220 is tubular, the distal part of control component 220 is linear, and the linear distal end of the control component 220 is disposed at a terminal or the outer surface of the tubular distal end of the control component 220. The proximal end of the blade 320 is accommodated in the proximal end of the control component 220, whereby the distal end of the control component 220 is provided between the inner wall of the outer tube 100 and the blade 320. Alternatively, the control component 220 is linear and disposed axially along the gap between the inner wall of the outer tube 100 and the blade 320, whereby the control component 220 is arranged between the inner wall of the outer tube 100 and the blade 320.

Furthermore, in this embodiment, the cutting component is the cutting head 310, which achieves cutting and separation of the captured valve clip from at least part of the native valve tissue held by the valve clip through the cutting edge of the cutting head 310. In this embodiment, the number of the cutting head 310 is one, preferably ring-shaped. In another embodiment, there are multiple cutting heads provided circumferentially around the axis of the outer tube 100. This embodiment does not have special restrictions on the shape of the blade of the cutting head 310, such as flat mouthed, serrated, straight toothed, or "I" shaped.

Furthermore, a radiopaque marker(s) is provided on the cutting head 310 to show the position of the cutting head 310.

In this embodiment, the distal end of the blade 320 is connected to the cutting head 310, and the proximal end of the blade 320 is connected to the cutting handle 330. Preferably, the cutting head 310, blade 320, and cutting handle 330 can be formed integrally. In an alternative embodiment, the cutting head 310, the blade 320, and the cutting handle 330 are independent and connected as one unit. In another alternative embodiment, any two of the cutting head 310, blade 320, and cutting handle 330 are formed in one piece and connected to the remaining third component. Furthermore, the cutting head 310 and the distal part of the blade 320 of the cutter 300 are disposed around the inner wall the retrieve cavity 110 of the outer tube 100, that is, around the inner side of the retrieve cavity, for better removal of the valve clip in the retrieve cavity of the outer tube. The cutting handle 330 can control the axial and circumferential movement of the cutting head 310 and blade 320 along the outer tube 100 to remove the valve clip. Here, the blade 320 is preferably a rod-shaped or tubular piece and made of biocompatible metal or polymer compound. For example, the blade 320 is made of stainless-steel tube or nickel titanium tube.

When using the valve clip removal device of the present disclosure to remove the valve clip, the capture head 210 is controlled by the capture handle 230 via the control component 220. Therefore, the capture head 210 can extend in a direction far away from the outer tube 100 and capture the valve clip. The capture handle 230 can also control the capture head 210 to make it retract axially along the outer tube 100 towards the proximal end of the outer tube 100, thereby taking the captured valve clip into the retrieve cavity 110 of the outer tube 100. Then, the cutting head 310 of the cutter 300 disposed around the inner wall of the retrieve cavity 110 of the outer tube 100 cuts off the captured valve clip in the retrieve cavity 110 of the outer tube 100. In the other word, the capture head 210 has a first state and a second state. In the first state, the capture head 210 is close to the distal end of the outer tube 100, facilitating the entry and exit of the removal device from the target position. On the other hand, the capture head 210 can provide support (a backstop) when cutting off the valve clip, facilitating the cutting off of the valve clip. In the second state, the capture head is far away from the distal end of the outer tube 100, and its function is to capture the valve clip. Furthermore, when cutting off the valve clip, the cutting head 320 can completely cut off the captured valve clip from the valve leaflet(s) (such as the anterior and posterior leaflet of the mitral valve) it holds, and the valve clip is no longer connected to the anterior and posterior leaflet. The valve clip is placed inside the retrieve cavity 110 and brought out of the body. Alternatively, the cutting head 320 cuts the captured valve clip away from one of the valve leaflets it holds, so that the valve clip is no longer connected to one valve leaflet (such as the anterior or posterior valve), but remains connected to the other valve leaflet. The valve clip remains at the valve and is not taken out of the body. The problem of opening caused by clipping the valve leaflets is resolved, so the valve prosthesis can be placed therein.

Furthermore, the capture head 210 is provided with a groove 211 on the outer side of the accommodating cavity 2101. The groove 211 is configured to accommodate the distal part of the cutting head 310 when the capture head 200 takes the captured valve clip into the retrieve cavity 110 of the outer tube 100, thereby a closed space is formed by the capture head 210 and the inner cavity of the outer tube 100.

Furthermore, in the axial direction of the outer tube 100, the capture handle 230 and the cutting handle 330 are provided far away from the distal end of the outer tube 100, and the capture handle 230 is provided above the cutting handle 330. Furthermore, a radiopaque marker(s) is provided at the distal end of the outer tube 100 to mark the position of the distal end of the outer tube.

In an alternative embodiment, the capture head 210 comprises a mesh structure or claw-like structure. For example, the capture head 210 includes a mesh structured capture net, which can first capture and fix the valve clip, and then pull the valve clip into the retrieve cavity 110 after the valve clip has been completely cut off from the valve leaflet(s) (such as the anterior leaflet and posterior leaflet of the mitral valve) it holds, or release the valve clip after cutting off the captured valve clip from one of the valve leaflets it holds. And for example, the capture head 210 includes a claw-like gripper, which can first capture and fix the valve clip, and then pull the valve clip into the retrieve cavity 110 after the valve clip has been completely cut off from the valve leaflet(s) (such as the anterior leaflet and posterior leaflet of the mitral valve) it holds, or release the valve clip after cutting off the captured valve clip from one of the valve leaflets it holds.

In an alternative embodiment, the cutting component comprises an electrode(s) electrically connected to a high-frequency AC power supply, and the electrode is needle shaped or blade shaped and is configured to separate the captured valve clip from at least part of the native valve tissue held by the valve clip by electrocoagulation. In another alternative embodiment, the cutting component comprises an electric heating wire(s) electrically connected to the power supply, and the electric heating wire is configured to separate the captured valve clip from at least part of the native valve tissue held by the valve clip by burning.

### Operating instructions for the valve clip removal device in an embodiment of the present disclosure:

The valve clip removal device in an embodiment of the present disclosure can enter the left atrium via the transapical approach. When approaching the valve clip, the capture head is released by operating the capture handle to control the control component. When the capture head catches the valve clip, operate the capture handle again to retract the capture head. At this point, the valve clip is pulled in the accommodating cavity (retrieve cavity) at the distal end of the outer tube. Then, operate the cutting handle to drive the blade and cutting head forward in a spiral manner along the axial and circumferential direction of the outer tube, until the cutting head contacts the tissue held by the valve clip, and continue to operate the cutting handle until the valve clip is cut off. After resection is completed, retract the cutting head toward the direction opposite to the cutting head enters.

### The main advantages of the valve clip removal device in some embodiments of the present disclosure are as follows:

(a) In some embodiments of the present disclosure, the valve clip removal device can enter the heart via the transapical approach and reach the vicinity of the native valve tissue, unlike other methods such as the transfemoral approach, which needs to add additional instruments such as guide catheter;
(b) In some embodiments of the present disclosure, the capture and removal of the valve clip by the valve clip removal device can be accomplished only through the outer tube and the capture device and the cutter inside the outer tube, eliminating the need for additional catheter(s), thus making the removal device simple and reliable in structure, convenient and quick in operation;
(c) In some embodiments of the present disclosure, the structure of the capture device of the valve clip removal device is ingenious, simple and reliable, making it easy for operators to operate quickly and conveniently. The structure of the capture head of the capture device can smoothly grasp the valve clip and provide support when cutting off the valve clip;
(d) The structure of the cutter of the valve clip removal device in some embodiments of the present disclosure is simple and reliable, and there is no need to introduce new cutter to operate the removal device;
(e) In some embodiments of the present disclosure, the cutting component of the valve clip removal device is disposed in the retrieve cavity of the outer tube for cutting, so that the cutting component will not be affected by other tissues, organs or blood flow;
(f) The cutting process of the valve clip removal device in some embodiments of the present disclosure is carried out in the retrieve cavity, which can prevent the tissue debris generated from cutting from flowing into the circulatory system and causing embolism.

All documents mentioned in this application are considered to be included as a whole in the public content of this application, so as to serve as a basis for modification if necessary. In addition, it should be understood that after reading the above disclosed content of this application, those skilled in the art may make various changes or modifications to this application, and these equivalent forms also fall within the scope of protection claimed by this application.

## Claims

1. A valve clip removal device, comprising: an outer tube (100), a capture device (200) and a cutter (300), wherein the capture device (200) comprises one or more capture heads (210) provided at a distal end, and the cutter (300) comprises a cutting component provided at a distal end;
wherein the outer tube (100) has a retrieve cavity (110), the capture heads (210) are movably provided at a distal end of the outer tube (100), and the capture heads (210) are configured to capture a valve clip; the cutting component is movably disposed in a cavity of the retrieve cavity (110) of the outer tube (100), and configured to separate a captured valve clip from at least part of a native valve tissue held by the valve clip.

2. The valve clip removal device according to claim 1, wherein the capture device (200) further comprises a control component (220) and a capture handle (230), the control component (220) passes through the retrieve cavity (110) of the outer tube (100), a distal end of the control component (220) is connected to the capture heads (210), a proximal end of the control component (220) is connected to the capture handle (230), and the capture handle (230) drives the capture heads (210) to move by controlling the control component (220), thereby achieving capture of the valve clip.

3. The valve clip removal device according to claim 1, wherein a proximal end of the capture head (210) comprises an accommodating cavity (2101), which is configured to accommodate the valve clip to achieve capture of the valve clip, and to retrieve the valve clip to be cut off into the retrieve cavity (110).

4. The valve clip removal device according to claim 3, wherein the capture head (210) is hemispheric, 1/4 spherical, pyramidal, cylindrical, conical or tetrahedral in shape.

5. The valve clip removal device according to claim 3, wherein the cutter (300) further comprises a blade (320) and a cutting handle (330), a proximal end of the blade (320) is connected to the cutting handle (330), and a distal end of the blade (320) is connected to the cutting component, the cutting handle (330) is configured to control the movement of the cutting component and the blade (320) relative to the outer tube (100), so as to separate the captured valve clip from at least part of the native valve tissue held by the valve clip.

6. The valve clip removal device according to claim 5, wherein at least a distal end of the control component (220) is disposed between a sidewall of the retrieve cavity (110) and the blade (320) in the radial direction.

7. The valve clip removal device according to claim 5, wherein the cutting component comprises a cutting head (310) for cutting and separating the captured valve clip from at least part of the native valve tissue held by the valve clip.

8. The valve clip removal device according to claim 7, wherein the cutting head (310) is circular in shape, or the cutting head (310) is in a plurality and arranged circumferentially around an axis of the outer tube (100).

9. The valve clip removal device according to claim 8, wherein the capture head (210) is provided with a groove (211) on an outer side of the accommodating cavity (2101), and wherein the groove (211) is configured to accommodate a distal part of the cutting head (310) to form a closed space by the capture head (210) and an inner cavity of the outer tube (100) when the capture head (200) takes the captured valve clip into the retrieve cavity (110) of the outer tube (100).

10. The valve clip removal device according to claim 1, wherein the cutting component comprises needle or blade shaped electrodes connected to a high-frequency AC power supply, the electrodes are configured to separate the captured valve clip from at least part of the native valve tissue held by the valve clip by electrocoagulation, or the cutting component comprises an electric heating wire electrically connected to a power supply, and the electric heating wire is configured to separate the captured valve clip from at least part of the native valve tissue held by the valve clip by burning.

11. The valve clip removal device according to any one of claims 1-10, wherein the proximal end of the capture head (210) is matched to the distal end of the outer tube (100) so as to form a closed space by the capture head (210) and the retrieve cavity (110) of the outer tube (100) when the capture head (210) takes the captured valve clip into the retrieve cavity (110) of the outer tube (100).

12. The valve clip removal device according to any one of claims 1-10, wherein the capture head (210) is provided with a radiopaque marker(s) for marking the position of the capture head (210).

13. The valve clip removal device according to any one of claims 1-10, wherein the distal end of the outer tube (100) is provided with a radiopaque marker(s) for marking the position of the distal end of the outer tube (100).

14. The valve clip removal device according to any one of claims 1-10, wherein the capture head (210) comprises a mesh structure or a claw-like structure for capturing and fixing the valve clip.
